# EUROPEAN PATENT SPECIFICATION

(11) **EP 1 443 899 B1**
(45) Date of publication and mention of the grant of the patent: **18.08.2010**
(21) Application number: 02770290.1
(22) Date of filing: 29.10.2002
(51) Int. Cl.: A61K 9/00

(54) **BOWEL CLEANSING AGENT**
LÖSUNG ZUR DARMREINIGUNG
AGENT DE NETTOYAGE INTESTINAL

(30) Priority: 29.10.2001 JP 2001330626; 29.10.2001 JP 2001330627
(43) Date of publication of application: 11.08.2004
(73) Proprietor: Ajinomoto Co., Inc., Chuo-ku, Tokyo 104-8315 (JP)
(72) Inventor: SUGIYAMA, Takayuki, Shimizu-shi, Shizuoka 424-0945 (JP); SUZUKI, Mayumi, Rockville, MD 20852 (US)
(74) Representative: Albrecht, Thomas
(86) International application number: PCT/JP2002/011188
(87) International publication number: WO 2003/037298

(56) References cited:
- KATO T ET AL: "Screening for colon cancer in patients with schizophrenia" JAPANESE PHARMACOLOGY AND THERAPEUTICS 1995 JAPAN, vol. 23, no. 12, 1995, pages 243-246, XP001146539 ISSN: 0386-3603
- YAMAMOTO M ET AL: "Combination preparation with PEG-ELS 1000 ml and Magcorol P50g in colonoscopic studies" THERAPEUTIC RESEARCH 1993 JAPAN, vol. 14, no. SUPPL. 2, 1993, pages 197-199, XP001146540 ISSN: 0289-8020
- YEE JUDY ET AL: "Colorectal neoplasia: Performance characteristics of CT colonography for detection in 300 patients." RADIOLOGY, vol. 219, no. 3, June 2001 (2001-06), pages 685-692, XP009008184 ISSN: 0033-8419
- CITTADINI G ET AL: "A NEW MAGNESIUM-CONTAINING PEG-ELECTROLYTE SOLUTION FOR THE ORAL LAVAGE OF THE COLON" CLINICAL RADIOLOGY, LIVINGSTONE, HARLOW, GB, vol. 54, no. 3, 1 March 1999 (1999-03-01), pages 160-163, XP009008103 ISSN: 0009-9260

## Description

### Technical Field

The present invention relates to a bowel cleansing preparation, which is useful in the pretreatment prior to colonoscopy or colonic surgery.

### Background Art

Heretofore, there have been known the bowel cleansing preparation to be taken prior to colonoscopy, colonic surgery and so on. These preparations are, for example: the preparation comprised of polyethylene glycol 4,000 (PEG 4,000) as water-soluble polymer and electrolytes (Gastroenterology 84, 1512, 1983; International Publication No. WO 87/00754; Japanese Patent Laid-open Hei 1-125319; Japanese Patent Laid-open Hei 1-132527; Japanese Patent Laid-open Hei 2-292223); the preparation comprised of PEG 4,000 or mannitol as water-soluble polymer and electrolytes (Gastroenterology 78, 991, 1980); the preparation comprised of PEG 4,000 and xylose as water-soluble polymer and electrolytes (Gastroenterology 79, 35, 1980); the preparation comprised of a water-soluble polymer selected from the group consisting of PEG 4,000, dextran, dextrin, hydroxylethyl starch, poly dextrose, gum arabic and/or pectin, and electrolytes (Japanese Patent Laid-open Hei 2-25424; Japanese Patent Laid-open Hei 3-206046); the preparation comprising of erythritol and/or xylitol as water-soluble polymer and electrolytes (Japanese Patent Laid-open Hei 3-284620); and the preparation using citric acid magnesium salt pentahydrate (Japanese Patent No. 3,047,143). In general, the patients have to take the conventional bowel cleansing preparations mentioned above in large dosage such as from 2 to 4 liters, with frequent nausea or vomiting and so on during the administration of some patients, and therefore, it is difficult to take such a large amount of the dosage. Accordingly, it has been desired to reduce the dosage of the preparation to be taken.

One objective of the present invention is to provide the bowel cleansing preparation having an excellent cleansing effect for intestinal tract without nausea or vomiting. The other objective of the present invention is to provide the bowel cleansing preparation, which can be taken in small amount without fullness and bloating, due to the superior cleansing effect for intestinal tract to the conventional bowel cleansing preparations.

To achieve these objectives, the present inventors have conducted the various studies with respect to the bowel cleansing preparation. As the results, they found out that favorable cleansing effect was brought by using the preparation containing at least one kind of ions selected from the group consisting of sulfate ion (SO₄²⁻) , phosphate ion (PO₄³⁻) and magnesium ion (Mg²⁺), and sodium ion, potassium ion, and bicarbonate ion, as the electrolyte commonly contained together with the water-soluble polymer. Particularly, the preparation exhibiting superior bowel cleansing effect can be obtained by additionally containing magnesium ion in the range of 0.1-3.0 g/dL, preferably 0.2-1.6 g/dL, as magnesium element in the preparation.

The same amount of electrolyte in the blood or intercellular fluid is contained in the conventional bowel cleansing preparation with the water-soluble polymer, and therefore magnesium ion is commonly contained in the preparation in the average amount of 3 mEq/L. However, the present invention is characterized by using the large amount of the magnesium ion in the preparation.

Further, the present inventors have conducted the studies on the water-soluble polymer contained in the bowel cleansing preparation. The water-soluble polymer used in the bowel cleansing preparation of the present invention is polyethylene glycol having a high average molecular weight, such as more than 5,000, and more specifically about 7,300 to about 9,300 (Macrogol ^{®} 6,000) compared to the common polyethylene glycol having an average molecular weight of about 2,600 to 3,800 (Macrogol^{®} 4,000). By using the polyethylene glycol having a high average molecular weight, the dosage of the preparation can be reduced in comparison to that of conventional bowel cleansing preparation. That is, the bowel cleansing effect of the preparation of the present invention can be achieved by administering in about half amount of dosage in comparison with the dosage of conventional bowel cleansing preparations.

The present inventions are completed by the findings of these studies.

### Disclosure of Invention

Accordingly, one essential aspect of the present invention is to provide the bowel cleansing preparation containing the water-soluble polymer and electrolytes of 30-150 mEq/L of sodium ion, 3-20 mEq/L of potassium ion, and 10-30 mEq/L of bicarbonate ion, wherein at least one kind of ions selected from the group consisting of sulfate ion, phosphate ion and magnesium ion is further contained as additional electrolyte.

The amount of sulfate ion, phosphate ion and magnesium ion contained in the bowel cleansing preparation of the present invention is 0.1-50 mEq/L of sulfate ion, 0.1-50 mEq/L of phosphate ion, and 0.1-3 g/dL of magnesium ion as magnesium element.

Of these ions, magnesium ion is preferably used and accordingly, a most preferred embodiment of the present invention is to provide the bowel cleansing preparation containing the water-soluble polymer and 30-150 mEq/L of sodium ion, 3-20 mEq/L of potassium ion and 10-30 mEq/L of bicarbonate ion, wherein 0.1-3 g/dL of magnesium ion as magnesium element is further contained.

For magnesium element, citric acid magnesium salt is used so as to contain magnesium ion for electrolyte of the present invention. Therefore, the present invention provides the bowel cleansing preparation containing citric acid magnesium salt for providing magnesium ion.

Another essential aspect of the present invention is to provide the bowel cleansing preparation containing a water-soluble polymer and electrolytes, wherein polyethylene glycol having an average molecular weight of about 5,000 and over is used as the water-soluble polymer.

In one specific embodiment, polyethylene glycol having an average molecular weight of about 7,300 to about 9,300 is preferably used, and the amount of water-soluble polymer is in the range of from 10 to 160 g/L.

Accordingly, a most preferred embodiment of the present invention is to provide the bowel cleansing preparation containing 10-160 g/L of polyethylene glycol having an average molecular weight of about 7,300 to about 9,300 as the water-soluble polymer, and 30-150 mEq/L of sodium ion, 3-20 mEq/L of potassium ion and 10-30 mEq/L of bicarbonate ion, wherein 0.1-3 g/dL of magnesium ion as magnesium element is further contained.

It is possible to further contain an antifoaming agent in the bowel cleansing preparation of the present invention to prevent the bubbling during the process of preparing or administering the preparation of the present invention. Therefore, another embodiment of the present invention is to provide the bowel cleansing preparation containing the antifoaming agent.

### Best Mode for Carrying Out the Invention

The feature of the bowel cleansing preparation of the present invention is containing not only sodium ion, potassium ion and bicarbonate ion, but also at least one kind of ions selected from the group consisting of sulfate ion, phosphate ion and magnesium ion.

The amount of the electrolytes of sodium ion, potassium ion, and bicarbonate ion are 30-150 mEq/L, 3-20 mEq/L, and 10-30 mEq/L, respectively.

In the present invention, by further containing at least one kind of ions selected from the group consisting of sulfate ion, phosphate ion and magnesium ion, the bowel cleansing preparation of the present invention has superior bowel cleansing effect to the conventional preparations.

These ions can be contained in the present preparation in the form of salt commonly used in this field. The salt is, for example, sodium salt, potassium salt and magnesium salt for sulfate ion and phosphate ion; citric salt, chloride salt, sulfuric salt, phosphoric salt and bicarbonic salt for magnesium ion.

For magnesium ion, citric acid magnesium salt is preferably used as the source of magnesium ion, and among them, magnesium citrate anhydrate and magnesium hydrogen citrate pentahydrate are more preferably used as the source of magnesium ion.

Although a certain ion contained in the preparation of the present invention can improve the bowel cleansing effect, several ions show much greater improvement in bowel cleansing effect when combined and contained together in the preparation.

The preferable amounts of these ions are 0.1-50 mEq/L of sulfate ion, 0.1-50 mEq/L of phosphate ion, and 0.1-3 g/dL of magnesium ion as magnesium element. In the case of using less than 0.1 g/dL of magnesium ion as magnesium element, it is difficult to obtain the desired bowel cleansing effect. On the other hand, in the case of using more than 3 g/dL of magnesium ion as magnesium element, the isotonic bowel cleansing preparation cannot be obtained.

The preferable preparation of the present invention contains magnesium ion as citric acid magnesium salt, and in particular 1.5-20 g/dL of magnesium citrate anhydrous or magnesium hydrogen citrate pentahydrate is preferably contained. Therefore, one of specific features of the present invention is the bowel cleansing preparation containing a large amount of magnesium ion.

The water-soluble polymer to be used in the preparation of the present invention is, for example, polyethylene glycol (Macrogol^{®}), poly dextrose, hydroxylethyl starch, dextran, dextrin, gum arabic, pullulan, pectin, carboxymethyl cellulose, and so on. Among them, polyethylene glycol is preferably used as water-soluble polymer for the bowel cleansing preparation of the present invention, and especially, polyethylene glycol having a high average molecular weight, such as about 5,000 and over, more specifically about 7,300 to about 9,300 (Macrogol^{®} 6,000) compared to the common polyethylene glycol having an average molecular weight of about 2,600 to 3,800 (Macrogol^{®} 4,000) is most preferably used. The excellent bowel cleansing effect of the present invention can be obtained by using the polyethylene glycol having a high average molecular weight, and in administering about half times as large as the conventional bowel cleansing preparation in volume. The polyethylene glycol having higher molecular weight than Macrogol^{®} 6,000 can be also used in the present invention, and the use of such a high molecular polyethylene glycol falls within the scope of the present invention.

In the bowel cleansing preparation of the present invention, the amount of water-soluble polymer is preferably in the range of from 10 to 160 g/L.

The bowel cleansing preparation of the present invention described above can contain further additives, which prevent the flatulence by enteric bacterium. To be easy to take the preparation all the more, the additives may be perfume material such as citrus fragrance and flavoring substance such as sodium saccharin.

Further, it is preferable to contain an antifoaming agent in order to solve the bubbling problems. By using polyethylene glycol having high molecular weight, the bubbling during the preparation or administration of the present invention is remarkably removed, and therefore, it is preferable to contain the antifoaming agent in the preparation to prevent the bubbling. The antifoaming agent to be contained in the preparation may be clinically available agent such as a silicon antifoaming agent.

The bowel cleansing preparation of the present invention may be prepared by mixing the predetermined amount of the water-soluble polymer and electrolytes, and by adding the efficient amount of water and other additives if desired. Then the resulting mixture is well mixed to obtain the liquid form of the preparation of the present invention. Further, the preparation of the present invention may convert to the solid preparation such as powdery form, granules, or fine granules by drying the liquid preparation obtained above.

The liquid preparation may be filled into glass vial bottle or polymer container such as a plastic bag. The bowel cleansing preparation of the present invention may be prepared as the condensed form or powdery form in order to prepare the preparation in site. The condensed form or powdery form of the preparation of the present invention contains the amount of the dosage of single administration, and is diluted with water, preferably with distilled water to obtain the administrable preparation.

The dosage of the preparation of the present invention may depend on the patients to be administered, and in general, 1-2 liters of the preparation is applied for single administration. The preparation may be administered orally in general; however, nasal or rectal route may be available.

### Examples

The present invention will now be described in detail with examples or test examples.

### Example 1:

29.5 g of Macrogol^{®} 6,000, 18.9 g of magnesium hydrogen citrate pentahydrate, 0.843 g of sodium bicarbonate, 1.17 g of anhydrous sodium sulfate and 0.198 g of potassium chloride were dissolved in the distilled water for injection to prepare 500 ml of solution of the present invention.

At the same time, 29.5 g of Macrogol^{®} 4,000, 0.733 g of sodium chloride, 0.371 g of potassium chloride, 2.84 g of anhydrous sodium sulfate and 0.843 g of sodium bicarbonate were dissolved in the distilled water for injection to prepare 500 ml of the comparative solution.

The non-fasting rats were divided into once administered group to 5 times administered group (20, 40, 60, 80 and 100 mL/kg groups) and orally administered 20 mL/kg of the solution of the present invention or the comparative solution prepared above, one to five times every 10 minutes, respectively.

At 3 hours after the first administration of the solution, the rats were laparotomized under ether anesthesia to expose the intestinal tract. The colon (from end of appendix to rectum) was isolated after clipped by clamp so that the content was not spilt out, and the content was collected on the petri dish, and dried over 2 hours at 50 °C.

The number of the rats evacuated watery diarrhea within 3 hours after administration was significantly larger in the group administered the solution of the present invention than the group administered the comparative solution.

### Example 2:

The following test solutions were prepared.

### Test Solution A:

118 g of Macrogol^{®} 4,000, 2.93 g of sodium chloride, 1.49 g of potassium chloride, 11.4 g of anhydrous sodium sulfate and 3.37 g of sodium hydrogen carbonate were mixed and 1,000 ml of water was added to this mixture. The resulting solution was mixed by turning the mixture, and observed whether the bubbling phenomenon of the solution was induced. Then the solution was added water to obtain the isotonic solution of 2,000 ml as the Test Solution A.

### Test Solution B:

118 g of Macrogol^{®} 6,000, 2.93 g of sodium chloride, 1.49 g of potassium chloride, 11.4 g of anhydrous sodium sulfate and 3.37 g of sodium bicarbonate were mixed and 1,000 ml of water was added to this mixture. The resulting solution was mixed by turning the mixture, and observed whether the bubbling phenomenon was induced. Then the solution was added water to obtain the isotonic solution of 1,700 ml as the Test Solution B.

### Test Solution C:

118 g of Macrogol^{®} 6,000, 2.93 g of sodium chloride, 1.49 g of potassium chloride, 11.4 g of anhydrous sodium sulfate, 3.37 g of sodium bicarbonate and 100 mg of poly(dimethylsiloxane) as the antifoaming agent were mixed and 1,000 ml of water was added to this mixture. The resulting solution was mixed by turning the mixture, and observed whether the bubbling phenomenon was induced. Then the solution was added water to obtain the isotonic solution of 1,700 ml as the Test Solution C.

Since the bubbling phenomenon of the Test Solution B was frequently observed in comparison with the bubbling phenomenon of the Test Solution A, in consequence, it took longer time to prepare the Test solution B than the Test Solution A. On the contrary, the bubbling phenomenon of the Test Solution C was significantly decreased compared to the bubbling phenomenon of the Test Solution B, and the effect of the antifoaming agent was confirmed.

Further, 10 mL/kg of the Test Solutions B and C were administered nasally to dogs through feeding tube every 10 minutes and the administration was discontinued at the point of the transparent feces was observed. The colon was examined endoscopically, and as the results, low visibility of the inside of colon was observed due to the bubbling phenomenon in the intestine tract in the dogs administered the Test Solutions B without the antifoaming agent. On the contrary, good visibility of the inside of colon was observed in the dogs administered the Test Solutions C containing the antifoaming agent, and thus the effect of the antifoaming agent was confirmed.

### Example 3:

29.5 g of Macrogol^{®} 4,000, 18.9 g of magnesium hydrogen citrate pentahydrate, 0.843 g of sodium bicarbonate, 0.913 g of citric acid trisodium salt dehydrate and 0.211 g of citric acid tripotassium salt monohydrate were dissolved in the distilled water to prepare 500 ml of the solution of the present invention.

29.5 g of Macrogol^{®} 4,000, 0.733 g of sodium chloride, 0.371 g of potassium chloride, 2.84 g of anhydrous sodium sulfate and 0.843 g of sodium bicarbonate were dissolved in the distilled water to prepare 500 ml of the comparative solution.

The non-fasting rats were divided into once administered group to 5 times administered group (20, 40, 60, 80 and 100 mL/kg groups) and orally administered 20 mL/kg of the solution of the present invention or the comparative solution prepared above, one to five times every 10 minutes, respectively.

At 3 hours after the first administration of the solution, the rats were laparotomized under ether anesthesia to expose the intestinal tract. The colon (from end of appendix to rectum) was isolated after clipped by clamp so that the content was not spilt out, and the content was collected on the petri dish, and dried over 2 hours at 50 °C.

The number of the rats evacuated watery diarrhea within 3 hours after administration was significantly larger in the group administered the solution of the present invention than the group administered the comparative solution.

### Example 4:

60 g of Macrogol^{®} 6,000, 18.7 g of magnesium hydrogen citrate pentahydrate, 1.79 g of sodium bicarbonate, 6.5 g of citric acid, trisodium salt dihydrate, and 0.9 g of citric acid, tripotassium salt monohydrate were dissolved in the distilled water to prepare 1,000 ml of the solution of the present invention.

As the comparative solution, the commercially available bowel cleansing preparation, Magcorol^{®} P (Horii Pharm. Ind., Ltd.), was used.

The bowel cleansing effect of the solution of the present invention was compared to those of the comparative solution in 6 healthy adult male volunteers.

Each volunteer took a meal of brief wheat noodle for the supper on the day before the test and suspension of 10 g of Magcorol^{®} P with small amount of water was administered orally at 9 p.m.

On the testing day, each volunteer abstained from breakfast. At 8 a.m. volunteers started to take 750 mL of the solution of the present invention or the comparative solution at a rate of 250 mL/15 minutes over 45 minutes. Then, the time of bowel movement initiating and time of changing to evacuate watery diarrhea were determined up to 6 hours after the administration.

3 volunteers took the solution of the present invention in the first week, and the comparative solution in the next week, and other 3 volunteers took the comparative solution in the first week, and the solution of the present invention in the next week.

The results were summarized in Tables 1 and 2.

**Table 1: The Condition of Evacuation After Taking the Solution of The Present Invention**

| Volunteer No. | Time for Initiating Evacuation (minutes) | Condition of Evacuation | Time for Changing to Watery Diarrea (minutes) |
|---|---|---|---|
| 1 | 45 | diarrheal stool | 87 |
| 2 | 57 | loose stool | 93 |
| 3 | 38 | diarrheal stool | 68 |
| 4 | 40 | loose stool | 62 |
| 5 | 47 | loose stool | 74 |
| 6 | 63 | diarrheal stool | 91 |
| Mean±S.D. | 48.5±9.79 | - | 79.1±13.0 |

**Table 2: The Condition of Evacuation After Taking the Comparative Solution**

| Volunteer No. | Time for Initiating Evacuation (minutes) | Condition of Evacuation | Time for Changing to Watery Diarrea (minutes) |
|---|---|---|---|
| 1 | 92 | loose stool | Not changed |
| 2 | Not Evacuated | - | - |
| 3 | 75 | loose stool | Not changed |
| 4 | 125 | loose stool | Not changed |
| 5 | 88 | diarrheal stool | Not changed |
| 6 | 116 | loose stool | Not changed |
| Mean±S.D. | 99.2±20.7* | - | - |

| | | | |
|---|---|---|---|
| *: Calculated without volunteer No. 2 | | | |

The volunteers administered the solution of the present invention were induced watery diarrhea or loose stool at 48.5 ± 9.79 minutes after the administration and watery diarrhea was observed at 79.1 ± 13.0 minutes after the administration. In contrast, by administration of the comparative solution, 5 volunteers were induced watery diarrhea or loose stool at 99.2 ± 20.7 minutes after the administration, and no evacuation was observed in 1 volunteer (No. 2). Further, no volunteer had watery diarrhea within 6 hours after the administration of the solution of the present invention.

Nausea, vomiting, fullness and abdominal bloating were not observed in any of the volunteers after taking the solution of the present invention.

### Industrial Applicability

As set forth, the bowel cleansing preparation of the present invention has superior bowel cleansing effect, and is useful in the pretreatment prior to colonoscopy or colonic surgery because the preparation shows sufficient effect even in small volume in comparison to the conventional bowel cleansing preparation in small volume. Therefore, the bowel cleansing preparation of the present invention can remarkably reduce the burden of the patients.

## Claims

1. A bowel cleansing preparation containing water-soluble polymer and electrolytes, wherein 0.1-3 g/dL of magnesium ion as magnesium element is contained, and wherein citric acid magnesium salt is contained as magnesium ions.

2. The bowel cleansing preparation according to claim 1, wherein 30-150 mEq/L of sodium ion, 3-20 mEq/L of potassium ion, and 10-30 mEq/L of bicarbonate ion are contained as electrolytes.

3. The bowel cleansing preparation according to claims 1 and 2, wherein polyethylene glycol having an average molecular weight of 5,000 and over is used as the water-soluble polymer.

4. The bowel cleansing preparation according to claim 3, wherein polyethylene glycol having an average molecular weight of 7,300 to 9,300 is used as the water-soluble polymer.

5. The bowel cleansing preparation claimed in any one of claims 1 to 4, wherein an antifoaming agent is further contained.

6. The bowel cleansing preparation claimed in any one of claims 1 to 5, wherein 0.2-1.6 g/dL of magnesium ion as magnesium element is contained.

7. A bowel cleansing preparation containing from 10 to 160 g/L of polyethylene glycol having an average molecular weight of 7,300 to 9,300 as the water-soluble polymer and electrolytes of 30-150 mEq/L of sodium ion, 3-20 mEq/L of potassium ion, 10-30 mEq/L of bicarbonate ion, and 0.1-3 g/dL of magnesium ion as magnesium element, wherein citric acid magnesium salt is contained as magnesium ions.

8. The bowel cleansing preparation claimed in any one of claims 1 to 7, in which the preparation is powdery form for use in the solution preparation at the time of administration thereof.

## Patentansprüche

1. Darmreinigungspräparat, das wasserlösliches Polymer und Elektrolyte enthält, in dem 0,1-3 g/dL Magnesiumion als Magnesiumelement enthalten sind und in dem Citronensäuremagnesiumsalz als Magnesiumionen enthalten ist.

2. Darmreinigungspräparat nach Anspruch 1, in dem 30-150 mEq/L Natriumion, 3-20 mEq/L Kaliumion und 10-30 mEq/L Bicarbonation als Elektrolyte enthalten sind.

3. Darmreinigungspräparat nach den Ansprüchen 1 und 2, in dem Polyethylenglykol mit einem durchschnittlichen Molekulargewicht von 5.000 und darüber als das wasserlösliche Polymer verwendet wird.

4. Darmreinigungspräparat nach Anspruch 3, in dem Polyethylenglykol mit einem durchschnittlichen Molekulargewicht von 7.300 bis 9.300 als das wasserlösliche Polymer verwendet wird.

5. Darmreinigungspräparat nach einem der Ansprüche 1 bis 4, in dem außerdem ein Antischaummittel enthalten ist.

6. Darmreinigungspräparat nach einem der Ansprüche 1 bis 5, in dem 0,2-1,6 g/dL Magnesiumion als Magnesiumelement enthalten sind.

7. Darmreinigungspräparat, enthaltend 10 bis 160 g/L Polyethylenglykol mit einem durchschnittlichen Molekulargewicht von 7.300 bis 9.300 als das wasserlösliche Polymer und Elektrolyte mit 30-150 mEq/L Natriumion, 3-20 mEq/L Kaliumion, 10-30 mEq/L Bicarbonation und 0,1-3 g/dL Magnesiumion als Magnesiumelement, in der Citronensäuremagnesiumsalz als Magnesiumionen enthalten ist.

8. Darmreinigungspräparat nach einem der Ansprüche 1 bis 7, wobei das Präparat in Pulverform zur Verwendung bei der Lösungsherstellung zur Zeit der Verabreichung desselben ist.

## Revendications

1. Préparation de nettoyage intestinal contenant un polymère soluble à l'eau et des électrolytes, dans laquelle 0,1-3g/dL des ions magnésium sont contenus en tant qu'élément de magnésium et dans laquelle le sel de magnésium de l'acide citrique est contenu en tant qu'ions magnésium.

2. Préparation de nettoyage intestinal selon la revendication 1, qui contient 30-150 mEq/L d'ions sodium, 3-20 mEq/L d'ions potassium, et 10-30 mEq/L d'ions bicarbonate en tant qu'électrolytes.

3. Préparation de nettoyage intestinal selon les revendications 1 et 2, dans laquelle un polyéthylène glycol présentant une masse moléculaire moyenne égale et supérieure à 5000 est utilisé en tant que polymère soluble à l'eau.

4. Préparation de nettoyage intestinal selon la revendication 3, dans laquelle un polyéthylène glycol présentant une masse moléculaire moyenne comprise entre 7300 et 9300 est utilisé en tant que polymère soluble à l'eau.

5. Préparation de nettoyage intestinal selon l'une quelconque des revendications 1 à 4 contenant en outre un additif antimousse.

6. Préparation de nettoyage intestinal selon l'une quelconque des revendications 1 à 5, qui contient 0,2-1,6 g/dL d'ions magnésium en tant qu'élément de magnésium.

7. Préparation de nettoyage intestinal contenant 10 à 160g/L de polyéthylène glycol présentant une masse moléculaire moyenne comprise entre 7300 et 9300 en tant que polymère soluble à l'eau et des électrolytes comprenant 30-150 mEq/L d'ions sodium, 3-20 mEq/L d'ions potassium, 10-30 mEq/L d'ions bicarbonate, et 0,1-3g/L d'ions magnésium en tant qu'élément de magnésium, dans laquelle le sel de magnésium de l'acide citrique est contenu en tant qu'ions magnésium.

8. Préparation de nettoyage intestinal selon l'une quelconque des revendications 1 à 7, la préparation étant sous forme de poudre pour l'utilisation dans la fabrication d'une solution au temps de l'administration de celle-ci.
